# EUROPEAN PATENT APPLICATION

(11) **EP 4 800 699 A1**
(43) Date of publication of application: **02.09.2026**
(21) Application number: 26160252.8
(22) Date of filing: 24.02.2026
(51) Int. Cl.: G16H 20/40, A61M 1/36

(54) **PERSONALIZATION OF BLOOD PROCESSING PROCEDURES**

(30) Priority: 26.02.2025 US 202563763455 P
(71) Applicant: Fenwal, Inc., Lake Zurich, IL 60047 (US)
(72) Inventor: MAHER, Jeffrey R., Lake Zurich, 60047 (US); QUEZADA, Francesca S., Lake Zurich, 60047 (US); DICOLA, Nicholas R., Lake Zurich, 60047 (US); ANTUNA, Isaac G., Lake Zurich, 60047 (US)
(74) Representative: Maikowski & Ninnemann Patentanwälte Partnerschaft mbB

(57) **Abstract**

A blood processing device includes a pump system configured to be actuated during a blood processing procedure to draw blood from a subject and/or to convey a fluid to the subject. The blood processing device also includes a controller programmed to control operation of the pump system during the blood processing procedure. The controller is further programmed to retrieve or be provided with information regarding a medical profile of the subject, adjust at least one parameter of the blood processing procedure based at least in part on the medical profile of the subject, and execute the blood processing procedure with the adjusted parameter.

## Description

### Background

### Field of the Disclosure

The invention relates to blood processing procedures. More particularly, the invention relates to personalization of blood processing procedures based on the medical profile of a subject.

### Description of Related Art

Once blood has been drawn from a subject (e.g., a patient or a donor), the blood may be processed in any of a number of ways. For example, various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

According to one approach, whole blood may be separated into its constituents through centrifugation. This requires that the whole blood be passed through a centrifuge after it is withdrawn from, and before any constituent thereof is returned to, the blood source. To reduce contamination and possible infection (if the blood source is a human donor or patient), the blood is preferably processed within a sealed, sterile fluid flow circuit during the centrifugation process. The operator installs a fresh, sterile disposable flow circuit in the centrifuge before processing and removes and discards it afterwards. Typical disposable flow circuits are sealed and sterile, and include a separation chamber portion, which is mounted in cooperation on a durable, reusable assembly containing the hardware (centrifuge, drive system, pumps, valve actuators, programmable controller, and the like) that rotates the separation chamber and controls flow through the fluid circuit. The separation chamber may be formed of a generally rigid material (e.g., molded plastic), in which case the chamber itself defines a flow path or channel in which blood is separated into two or more components, or a more flexible material (e.g., in the form of a belt or annulus), which relies upon the system hardware to support the chamber and define the shape of the chamber as blood flows through it.

With a disposable circuit loaded onto the centrifuge (or just prior to or during loading) the operator typically enters, for example, by means of a touch screen or other user interface system, a particular processing protocol to be executed by the system (e.g., a procedure wherein platelets are separated from whole blood and collected) and other parameters (e.g., the weight of the blood source, the desired volume of separated blood component to be collected, etc.). When the system has been programmed, the operator fluidly connects the source to the circuit and the system carries out the procedure, under the supervision of the operator.

The centrifuge rotates the separation chamber of the disposable flow circuit during processing, causing the heavier (greater specific gravity) components of the whole blood in the separation chamber, such as red blood cells, to move radially outwardly away from the center of rotation toward the outer or "high-G" wall of the separation chamber. The lighter (lower specific gravity) components, such as plasma, migrate toward the inner or "low-G" wall of the separation chamber. The boundary that forms between the heavier and lighter components in the separation chamber is commonly referred to as the interface. Various ones of these components can be selectively removed from the whole blood by providing appropriately located channeling structures and outlet ports in the flow circuit. For example, in one blood separation procedure, plasma is separated from cellular blood components and collected, with the cellular blood components and a replacement fluid being returned to the blood source. Alternatively, red blood cells may be harvested from the separation chamber and the rest of the blood constituents returned to the donor. Other processes are also possible including, without limitation, platelet collection, red blood cell exchanges, plasma exchanges, etc.

While many blood separation systems and procedures have employed centrifugal separation principles, there are other classes of devices, including those based on the use of a spinning membrane. More specifically, this type of device employs relatively rotating surfaces, at least one of which carries a porous membrane. Typically, the device employs an outer stationary housing and an internal spinning rotor covered by a porous membrane. Blood is fed into an annular space or gap between the rotor and the housing. The blood moves along the longitudinal axis of the housing toward an exit region, with plasma passing through the membrane and out of the housing into a collection bag. The remaining blood components, primarily red blood cells, platelets, and white blood cells, move to the exit region between the rotor and the housing and then are returned to the donor or conveyed into a collection bag.

A system incorporating both a centrifuge and a spinning membrane separator is described in PCT Patent Application Publication No. WO 2018/053217 A1, which is hereby incorporated by reference herein. It should be understood that blood separation may be achieved by a variety of approaches, and that centrifugation and separation via spinning membrane are merely two exemplary approaches to blood separation. It should also be understood that certain blood processing procedures (such as whole blood donations) do not include separation of blood and return of a selected blood component to a subject, while others may include delivery of a fluid to the bloodstream of a subject without withdrawal of blood from the subject.

Depending on the particular steps that are executed in a blood processing procedure, there are various characteristics and/or preferences of a subject that may be relevant to execution of the procedure. For example, most blood processing procedures require anticoagulation of blood drawn from a subject in order to prevent a hazardous situation, such as blood clots or blood loss. For procedures in which anticoagulated blood is separated, followed by a separated blood component being returned to the blood source, some of the anticoagulant (which will tend to remain with the plasma of the blood following separation) will be delivered to the source. The anticoagulant may contain citrate, which binds to calcium and must be processed by the body. When citrate is delivered to a subject faster than the body of the subject can process it, citrate reactions may occur. Due to various medical conditions, certain subjects are particularly susceptible to citrate reactions at much lower levels than others, such that care should be taken when executing blood processing procedures for such subjects to ensure that citrate is delivered to them at a rate that their body can process so as to reduce the risk of such reactions.

Another consideration for many blood processing procedure is the rate at which blood is drawn from the subject, with the rate of fluid return being relevant for those procedures in which fluid is delivered to the subject. When a blood draw rate is too high, the vein of a subject may collapse, which can be painful to the subject. When a fluid return rate is too high, there is a chance of hematoma or chills. Suitable blood draw rates and fluid return rates will vary from subject to subject, so implementation of a standard version of a blood processing procedure (including a standard blood draw rate and/or a standard fluid return rate) may be uncomfortable or problematic for certain subjects.

Yet another consideration for many blood processing procedures is the volume of blood drawn from the subject during the procedure. Removing too much blood from a patient (hypovolemia) can cause fainting or other negative impacts. Additionally, if too much blood is drawn at any one time, any blood or separated blood components that is returned to the subject later in the procedure may be a relatively low temperature (due to being outside of the body for a longer amount of time), potentially causing chills in the subject. Suitable blood draw volume will vary from subject to subject, so implementation of a standard version of a blood processing procedure (including a standard volume of blood being drawn during a single cycle of the procedure) may be uncomfortable or problematic for certain subjects.

Other preferences or characteristics of a subject may also affect the parameters that are suitable for a blood processing procedure for that subject (such as the duration of the procedure) and even the types of procedures that are suitable for the subject. For example, for any of a number of reasons, a double red blood cell collection procedure (in which two units of red blood cells are collected from a donor) may be impracticable for certain subjects, with those subjects being limited to single red blood cell collection procedures and other blood processing procedures in which fewer than two units of red blood cells are collected. As another example, the platelets of certain subjects (who may be referred to as "aggregators") require resting after a procedure in order for the platelets to become eligible for storage/transfusion. For such subjects, a standard anticoagulant-to-whole blood ratio (which may be one part anticoagulant to eleven or twelve parts whole blood) may be too low, with an adjusted ratio (such as one part anticoagulant to seven parts whole blood) reducing the risk of platelet aggregation or the need for the platelets to be rested after a procedure.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a blood processing device is provided. The blood processing device includes a pump system configured to be actuated during a blood processing procedure to draw blood from a subject and/or to convey a fluid to the subject. The blood processing device also includes a controller programmed to control operation of the pump system during the blood processing procedure. The controller is further programmed to retrieve or be provided with information regarding a medical profile of the subject, adjust at least one parameter of the blood processing procedure based at least in part on the medical profile of the subject, and execute the blood processing procedure with the adjusted parameter.

### Brief Description of the Drawings

Fig. 1 is a is a perspective view of an exemplary blood processing device employing aspects of the present disclosure; and
Fig. 2 is a perspective view of an exemplary centrifugal separator of the blood processing device of Fig. 1.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

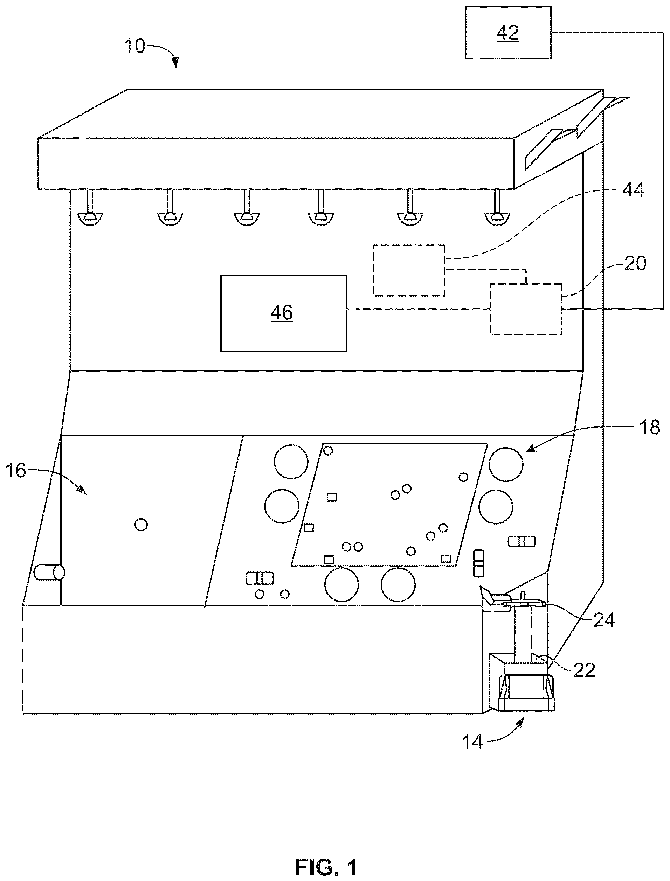

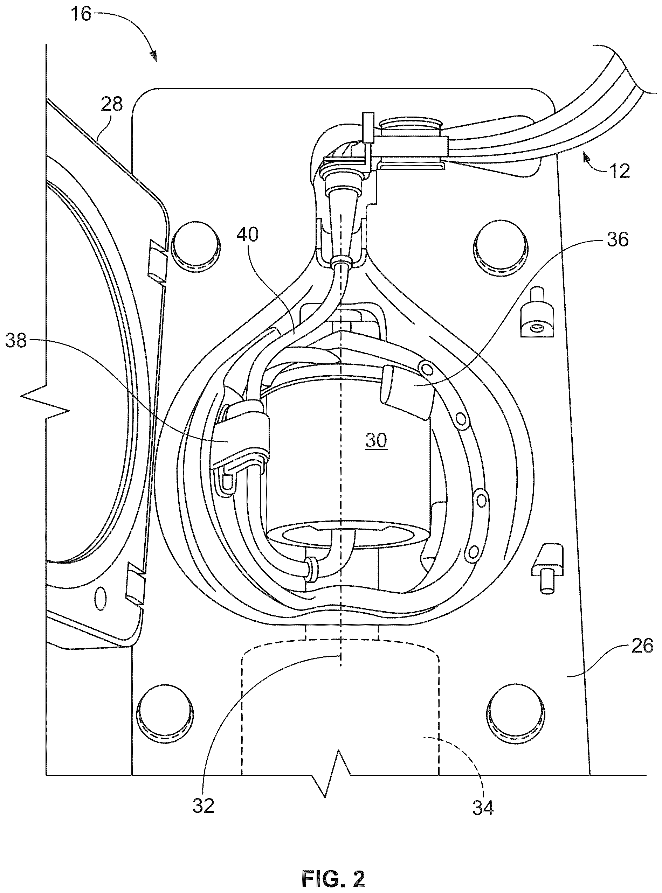

Systems according to the present disclosure will typically include two principal components - a durable or reusable blood processing device 10 (Fig. 1) and a disposable fluid flow circuit 12 (Fig. 2). It should be understood that the illustrated blood processing device 10 and fluid flow circuit 12 are merely exemplary and that the principles described herein may be practiced with a variety of differently configured blood processing devices and fluid flow circuits.

The illustrated blood processing device 10 includes a spinning membrane separator drive unit 14, a centrifuge or centrifugal separator 16 (Fig. 2), a pump system 18 and various valves that control fluid flow through the disposable flow circuit 12, and assorted other components (e.g., sensors, weight scales, etc.). The blood processing device 10 also includes a controller 20 that governs the operation of the other components of the blood processing device 10 to perform a blood processing procedure selected by an operator. The controller 20 may be variously configured without departing from the scope of the present disclosure. In one embodiment, the controller 20 may include a microprocessor (which, in fact may include multiple physical and/or virtual processors). According to other embodiments, the controller 20 may include one or more electrical circuits designed to carry out the actions described herein. In fact, the controller 20 may include a microprocessor and other circuits or circuitry. In addition, the controller 20 may include one or more memories. The instructions by which the microprocessor is programmed may be stored on the memory associated with the microprocessor, which memory/memories may include one or more tangible non-transitory computer readable memories, having computer executable instructions stored thereon, which when executed by the microprocessor, may cause the microprocessor to carry out one or more actions as described below.

The illustrated spinning membrane separator drive unit 14 is configured to accommodate a generally cylindrical spinning membrane separator of the fluid flow circuit 12. PCT Patent Application Publication No. WO 2012/125457 A1, which is hereby incorporated by reference herein, describes an exemplary spinning membrane separator that would be suitable for incorporation into the fluid flow circuit 12, but it should be understood that the spinning membrane separator and the matching spinning membrane separator drive unit 14 may be differently configured without departing from the scope of the present disclosure.

The spinning membrane separator drive unit 14 may include a base 22 configured to receive a lower portion of the spinning membrane separator and an upper end cap 24 to receive an upper portion of the spinning membrane separator. The upper end cap 24 may be positioned directly above the base 22 to orient a spinning membrane separator received by the spinning membrane separator drive unit 14 vertically and to define a vertical axis about which the spinning membrane separator is spun. While it may be advantageous for the spinning membrane separator drive unit 14 to vertically orient a spinning membrane separator, it is also within the scope of the present disclosure for the spinning membrane separator to be differently oriented when mounted to the blood processing device 10.

At least one of the base 22 and the upper end cap 24 is configured to spin one or more components of the spinning membrane separator about the axis defined by the spinning membrane separator drive unit 14. The mechanism by which the spinning membrane separator drive unit 14 spins one or more components of the spinning membrane separator may vary without departing from the scope of the present disclosure. In one embodiment, a component of the spinning membrane separator to be spun includes at least one element configured to be acted upon by a magnet (e.g., a metallic material), while the spinning membrane separator drive unit 14 includes a magnet (e.g., a series of magnetic coils or semi-circular arcs). By modulating the magnetic field acting upon the aforementioned element of the spinning membrane separator, the component or components of the spinning membrane separator may be made to spin in different directions and at varying speeds. In other embodiments, different mechanisms may be employed to spin the component or components of the spinning membrane separator.

Regardless of the mechanism by which the spinning membrane separator drive unit 14 spins the component or components of the spinning membrane separator, the component or components of the spinning membrane separator is/are preferably spun at a speed that is sufficient to create Taylor vortices in a gap between the spinning component and a stationary component of the spinning membrane separator (or a component that spins at a different speed). Fluid to be separated within the spinning membrane separator flows through this gap, and filtration may be dramatically improved by the creation of Taylor vortices.

Typically, a spinning membrane separator drive unit 14 and matching spinning membrane separator are used to separate plasma from cellular blood components. For example, in one embodiment blood enters a gap between an outer stationary housing and an internal spinning rotor covered by a porous membrane. The blood moves along the longitudinal axis of the housing toward an exit region, with plasma passing through the membrane and out of the housing into a collection bag. The remaining blood components, primarily red blood cells, platelets, and white blood cells, move to the exit region between the rotor and the housing and then are returned to the donor or conveyed into a collection bag. While it is most common for a spinning membrane separator drive unit 14 and matching spinning membrane separator to be used to separate plasma from other blood components, it is within the scope of the present disclosure for the combination (if provided) to separate other blood components.

As for the centrifugal separator 16, it includes a centrifuge compartment 26 that may receive the other components of the centrifugal separator 16 (Fig. 2). The centrifuge compartment 26 may include a lid 28 that is opened to insert and remove a centrifugal separation chamber 30 of the fluid flow circuit 12. During a blood processing procedure that includes a blood separation step, the lid 28 may be closed with the centrifugal separation chamber 30 positioned within the centrifuge compartment 26, as the centrifugal separation chamber 30 is spun or rotated about an axis 32 under the power of an electric drive motor or rotor 34 of the centrifugal separator 16.

The illustrated centrifugal separator 16 includes a carriage or support 36 that holds the centrifugal separation chamber 30 and a yoke member 38. The yoke member 38 engages an umbilicus 40 of the fluid flow circuit 12, which extends between the centrifugal separation chamber 30 and one or more other components (e.g., a cassette) of the fluid flow circuit 12. The yoke member 38 causes the umbilicus 40 to orbit around the centrifugal separation chamber 30 at a one omega rotational speed. The umbilicus 40 twists about its own axis as it orbits around the centrifugal separation chamber 30. The twisting of the umbilicus 40 about its axis as it rotates at one omega with the yoke member 38 imparts a two omega rotation to the centrifugal separation chamber 30, according to known design. The relative rotation of the yoke member 38 at a one omega rotational speed and the centrifugal separation chamber 30 at a two omega rotational speed keeps the umbilicus 40 untwisted, avoiding the need for rotating seals.

For blood processing procedures including a blood separation step, blood is introduced into the centrifugal separation chamber 30 by the umbilicus 40, with the blood being separated (e.g., into a layer of less dense components, such as platelet-rich plasma, and a layer of more dense components, such as packed red blood cells) within the centrifugal separation chamber 30 as a result of centrifugal forces as it rotates. Components of an interface monitoring system may be positioned within the centrifuge compartment 26 to oversee separation of blood within the centrifugal separation chamber 30, with the controller 20 adjusting operation of the various components of the blood processing device 10 in response to transmissions from the interface monitoring system. For example, the controller 20 may command the pump system 18 to operate differently in order to adjust the position of an interface between separated blood components within the centrifugal separation chamber 30.

The separated blood components are conveyed out of the centrifugal separation chamber 30 for collection or return to the blood source. If additional processing of one of the components is required, then it may be conveyed into a second separator (e.g., the spinning membrane separator drive unit 14) or back into the centrifugal separator 16. For example, if platelets are to be collected, the blood may be separated in a first compartment of the centrifugal separation chamber 30 into packed red blood cells and platelet-rich plasma. The packed red blood cells are conveyed out of the centrifugal separation chamber 30 for collection or return to the blood source, while the platelet-rich plasma is conveyed out of the first compartment of the centrifugal separation chamber 30 and into a second compartment of the centrifugal separation chamber 30. The platelet-rich plasma in the second compartment separates into platelet-poor plasma and platelet concentrate, with the plasma being conveyed out of the centrifugal separation chamber 30 while platelet concentrate remains in the second compartment. The platelet concentrate may remain in the second compartment of the centrifugal separation chamber 30 at the end of the procedure as a collected product or may be conveyed out of the second compartment for collection in a separate container.

While the illustrated blood processing device 10 includes two separators, it should be understood that a blood processing device according to the present disclosure may have only one separator (e.g., only a centrifugal separator or only a spinning membrane separator drive unit or a differently configured separator) or no separators. If two separators are provided, as in the illustrated embodiment, blood may be separated using only one of the separators or both separators. For example, if the centrifugal separation chamber 30 includes only one compartment, blood may be conveyed into the centrifugal separator 16, which separates the blood into two or more components (e.g., red blood cells and platelet-rich plasma). One of the components may be conveyed from the centrifugal separator 16 into the spinning membrane separator drive unit 14, where it is further separated (e.g., into platelet-poor plasma and platelet concentrate) or processed.

Regardless of the particular configuration of the blood processing device 10, the separator(s), and the controller 20, the controller 20 is configured and/or programmed to execute at least one blood processing procedure but, more advantageously, is configured and/or programmed to execute a variety of different blood processing procedures. In any of these procedures, it would be advantageous for the various parameters of the procedure to be personalized to the subject of the procedure so as to improve the comfort of the subject, reduce the risk of a procedure needing to be paused or canceled, and otherwise improve the results of the procedure (e.g., improving the yield of a target blood component to be collected from the blood of the subject). Thus, the controller 20 is programmed to retrieve or to be provided with information regarding the medical profile of a subject of a blood processing procedure to be executed using the blood processing device 10. Once the controller 20 is in possession of the medical profile of the subject, it may assess the various parameters of a procedure to be performed on the subject and proceed to execute the procedure with one or more procedural parameters that have been adjusted in view of the medical profile of the subject.

As used herein, the term "medical profile" is intended to encompass any information regarding the subject that may be relevant to a blood processing procedure. For example, the medical profile of a subject may include physical characteristics of the subject and/or the blood of the subject that could be relevant to a blood processing procedure. The physical characteristics of a subject may include, for example, the weight of the subject and the sex of the subject. The physical characteristics of the blood of the subject may include, for example, the hematocrit of the blood or whether it is lipemic or whether the platelets have a tendency to become clumped (i.e., if the subject is an "aggregator"). The medical profile of a subject may also include information regarding any medical conditions that the subject may have that could be relevant to a blood processing procedure, such as anemia or an increased risk of a citrate reaction. The medical profile of a subject may further include previously indicated preferences of the subject that could be relevant to a blood processing procedure, such as the duration of a procedure that they can tolerate, the maximum blood draw rate that the subject can comfortably tolerate, or the maximum fluid return rate that the subject can comfortably tolerate.

The medical profile of a subject may also include information regarding a previous iteration of the same type of blood processing procedure that is to be performed on the subject. This may include, for example, a response of the subject to a previous iteration of the same type of procedure, such as a feeling of faintness following completion of the procedure. This may also include, for example, a result of execution of a previous iteration of the same type of procedure for the subject, such as any difference between a target volume of a separated blood component to have been collected and the actual volume of the blood component that was collected or a difference between an expected or target concentration or number of cells collected and the actual concentration or number of cells collected. This may further include, for example, information regarding an adjustment made to a procedural parameter for a previous iteration of the same type of procedure for the subject, such as a change in the volume of blood drawn from the subject during a single cycle of the procedure, a change in the blood draw rate, a change in the fluid return rate, a change in the total volume of blood processed during the procedure, and a change in the target volume of a separated blood component to be collected, along with an indication as to whether the change to the procedural parameter was successful.

The medical profile of a subject may also include information regarding previous execution of a blood processing procedure for the subject that is a different type than the one to be performed on the subject (e.g., information regarding a whole blood donation previously made by a subject who is to be the subject of an infusion procedure in which no blood is to be drawn). This may include, for example, a response of the subject to a different type of procedure, such as a feeling of faintness following completion of the procedure. This may also include, for example, a result of execution of a different type of procedure for the subject, such as any difference between a target volume of a separated blood component to have been collected and the actual volume of the blood component that was collected. This may further include, for example, information regarding an adjustment made to a procedural parameter for a different type of procedure for the subject, such as a change in an anticoagulant ratio, along with an indication as to whether the change to the procedural parameter was successful.

The medical profile of a subject (or a portion of their medical profile) may be retrieved by the controller 20 from any suitable source or provided to the controller 20 from any suitable source, which may include the controller 20 retrieving information from a plurality of sources, being provided with information from a plurality of sources, and retrieving information from at least one source while being provided with information from at least one other source. In one exemplary embodiment, the controller 20 may be programmed to retrieve information regarding a subject from an external source, such as a data management system or blood establishment computer system 42. Alternatively, the controller 20 may be configured to be provided with information regarding a subject from a data management system or blood establishment computer system 42 or some other external source, without the controller 20 actively retrieving that information. In another embodiment, the controller 20 may be programmed to retrieve information regarding a subject from an internal source, such as a database 44 incorporated into the blood processing device 10. Alternatively, the controller 20 may be configured to be provided with information regarding a subject from a built-in database 44 or other internal source, without the controller 20 actively retrieving that information.

In yet another embodiment, the controller 20 may be configured to receive information regarding the medical profile of a subject from an operator of the blood processing device 10. The operator may provide such information to the controller 20 in any manner without departing from the scope of the present disclosure. For example, the blood processing device 10 may be provided with a display screen 46 that is coupled to the controller 20. In such an embodiment, the controller 20 may be programmed to control the display screen 46 to display a prompt for the operator to enter information regarding a subject, with the subject subsequently providing the controller 20 with any available information. The display screen 46 may be configured as a touch screen, in which case the display screen 46 may display a variety of icons that may be touched by the operator to provide information regarding a subject to the controller 20 using the display screen 46. For example, the display screen 46 may present an icon for the operator to press if a subject is prone to citrate reactions or an icon for the operator to indicate a maximum blood draw rate that a subject will comfortably tolerate.

Regardless of the manner in which the controller 20 obtains the medical profile of a subject, it considers the information regarding the subject and determines whether it would be appropriate to make an adjustment to any of the standard parameters of a procedure. By way of example, the types of procedural parameters that may be adjusted by the controller 20 in response to obtaining the medical profile of a subject include (but are not limited to): blood draw rate, fluid return rate, anticoagulant ratio, procedure duration, maximum extracorporeal blood volume, and maximum citrate return rate. It should be understood that these procedural parameters are merely exemplary and that the types of parameters of a procedure that may be adjusted by the controller 20 in response to obtaining the medical profile of a subject may vary without departing from the scope of the present disclosure.

The magnitude and nature of the adjustments made by the controller 20 prior to execution of a particular procedure may also vary without departing from the scope of the present disclosure and will depend on the needs and/or preferences of the subject. Blood draw rate may be adjusted, for example, when a subject has indicated discomfort resulting from blood being drawn at a certain rate during a previous procedure, with the controller 20 executing a subsequent procedure with a lowered blood draw rate. Similarly, fluid return rate may be adjusted, for example, when a subjected has indicated discomfort resulting from fluid being delivered to them at a certain rate during a previous procedure, with the controller 20 executing a subsequent procedure with a decreased fluid return rate. Anticoagulant ratio may be increased (resulting in more anticoagulant being used than is standard) by the controller 20 for a procedure when the medical profile of a subject indicates a tendency of the platelets of the subject to clump following a procedure, for example. Procedure duration may be decreased by the controller 20 when the medical profile of a subject indicates a preference for a procedure to not last longer than a certain amount of time, for example. Maximum extracorporeal blood volume may be reduced by the controller 20 for a procedure when the medical profile of a subject indicates that the subject has a history of feeling faint when a certain volume of blood has been removed from their body at any one time, for example. Maximum citrate return rate may be decreased by the controller 20 for a procedure when the medical profile of a subject indicates that the subject is more prone to citrate reactions than is typical, for example.

When the controller 20 has determined that one or more adjustments should be made to a procedural parameter, it may proceed to automatically implement the change(s) when executing the procedure or, instead, advise an operator of the proposed adjustment(s) (e.g., via the display screen 46) and execute the procedure with the adjusted parameter(s) only after the operator has approved of the adjustment(s). In one embodiment in which the controller 20 is programmed to execute a plurality of different types of blood processing procedures, the controller 20 may be programmed to determine that, based on information from their medical profile, a selected procedure is inappropriate for the subject and prevent the procedure from being executed or, alternatively, alert an operator of a possible risk if the procedure were to be implemented and require the operator to confirm that the controller 20 should proceed with the selected procedure.

Adjustments directly made to certain procedural parameters by the controller 20 (which may be referred to herein as "primary" or "direct" adjustments) will necessarily lead to adjustments being made to other aspects of a procedure (which may be referred to herein as "secondary" or "consequential" adjustments). While some of these consequential adjustments will not result in the procedure being impacted in a way that would be considered negative, other consequential adjustments may have negative impacts on other aspects of a procedure. For example, adjusting an anticoagulant ratio (as a direct adjustment) will require an adjustment to be made to the rate at which anticoagulant is added to blood from a subject (as a consequential adjustment), though such an adjustment would not typically be considered to be a negative impact. On the other hand, reducing a blood draw rate (as a direct adjustment) will tend to increase the duration of a procedure (as a consequential adjustment), which may be considered to be a negative impact.

When a possible adjustment to a procedural parameter would result in a negative impact, the controller 20 may consider any of a number of factors to determine how exactly to implement a procedure so as to achieve the optimal result. This may include the controller 20 determining a plurality of possible adjusted parameter configurations for executing a procedure and comparing them to determine which best achieves the goals of the operator or the facility associated with the device 10. For example, the comfort level of a subject may be a factor in determining which of a plurality of possible adjusted parameter configurations should be selected for implementing a procedure. Another possible factor is the time required to complete the procedure using each of the different adjusted parameter configurations. Yet another possible factor is how the overall operational efficiency of the facility (e.g., blood center) for which the blood processing procedure is to be executed is affected by each of the different adjusted parameter configurations. An additional possible factor is the needs or goals of a facility for which the blood processing procedure is to be executed, which may include the amounts of certain products (e.g., red blood cell products or platelet products) that the facility is attempting to produce. When multiple factors are to be considered by the controller 20, it may be programmed to assign different priority levels or weights to each factor and make a determination based on the priority or weight assigned to each factor.

Blood processing devices according to the present disclosure provide several advantages over conventional devices. Compared to conventional devices that enable an operator to adjust one or more procedural parameters prior to beginning a procedure, a specially programmed controller will be able to make the same adjustments more quickly and without the risk of operator error. Additionally, as explained above, directly adjusting one procedural parameter may require consequential adjustments to be made to one or more other parameters, which may be difficult for an operator to implement properly. Thus, by providing a controller that is programmed to assess and make the appropriate adjustments to any procedural parameters before implementing a procedure, the cognitive load on the operator is reduced, while the resulting comfort and safety of the subject are improved. The likelihood of a procedure being paused or terminated may also be reduced by a controller that specifically tailors the procedure to the medical profile of a subject, thereby increasing the efficiency and likelihood of success of the procedure.

### Aspects

Aspect 1. A blood processing device, comprising: a pump system configured to be actuated during a blood processing procedure to draw blood from a subject and/or to convey a fluid to the subject; and a controller programmed to control operation of the pump system during the blood processing procedure, wherein the controller is further programmed to retrieve or be provided with information regarding a medical profile of the subject, adjust at least one parameter of the blood processing procedure based at least in part on the medical profile of the subject, and execute the blood processing procedure with said at least one adjusted parameter.

Aspect 2. The blood processing device of Aspect 1, wherein the medical profile of the subject includes a physical characteristic of the subject and/or the blood of the subject.

Aspect 3. The blood processing device of any one of the preceding Aspects, wherein the medical profile of the subject includes a previously indicated preference of the subject.

Aspect 4. The blood processing device of any one of the preceding Aspects, wherein the medical profile of the subject includes information regarding a response of the subject to execution of a previous iteration of the blood processing procedure.

Aspect 5. The blood processing device of any one of the preceding Aspects, wherein the medical profile of the subject includes information regarding a result of execution of a previous iteration of the blood processing procedure for the subject.

Aspect 6. The blood processing device of any one of the preceding Aspects, wherein the medical profile of the subject includes information regarding an adjustment made to a procedural parameter for a previous iteration of the blood processing procedure executed for the subject.

Aspect 7. The blood processing device of any one of the preceding Aspects, wherein the medical profile of the subject includes information regarding a response of the subject to execution of a different type of blood processing procedure.

Aspect 8. The blood processing device of any one of the preceding Aspects, wherein the medical profile of the subject includes information regarding a result of execution of a different type of blood processing procedure for the subject.

Aspect 9. The blood processing device of any one of the preceding Aspects, wherein the medical profile of the subject includes information regarding an adjustment made to a procedural parameter for a different type of blood processing procedure executed for the subject.

Aspect 10. The blood processing device of any one of the preceding Aspects, wherein the controller is programmed to adjust said at least one parameter of the blood processing procedure directly based on the medical profile of the subject, adjust a second parameter of the blood processing procedure based on adjustment of said at least one parameter of the blood processing procedure, and execute the blood processing procedure with said adjusted parameters.

Aspect 11. The blood processing device of any one of the preceding Aspects, wherein the controller is programmed to retrieve said information regarding the medical profile of the subject from a data management system or a blood establishment computer system or to be provided with said information regarding the medical profile of the subject from the data management system or the blood establishment computer system.

Aspect 12. The blood processing device of any one of the preceding Aspects, further comprising a display screen, wherein the controller is programmed to control the display screen to display a prompt for an operator to enter said information regarding the medical profile of the subject.

Aspect 13. The blood processing device of any one of the preceding Aspects, wherein the controller is programmed to execute a plurality of different types of blood processing procedures, and prevent execution of at least one of said plurality of different types of blood processing procedures based on the medical profile of the subject.

Aspect 14. The blood processing device of any one of the preceding Aspects, wherein the controller is programmed to determine a plurality of possible adjusted parameter configurations for executing the blood processing procedure for the subject, select one of the possible adjusted parameter configurations based on a required time to complete the blood processing procedure for the subject, and execute the blood processing procedure with the adjusted parameter configuration that has been selected.

Aspect 15. The blood processing device of any one of the preceding Aspects, wherein the controller is programmed to determine a plurality of possible adjusted parameter configurations for executing the blood processing procedure for the subject, select one of the possible adjusted parameter configurations based on a comfort level for the subject during the blood processing procedure, and execute the blood processing procedure with the adjusted parameter configuration that has been selected.

Aspect 16. The blood processing device of any one of the preceding Aspects, wherein the controller is programmed to determine a plurality of possible adjusted parameter configurations for executing the blood processing procedure for the subject, select one of the possible adjusted parameter configurations based on an operational efficiency of a facility for which the blood processing procedure is to be executed, and execute the blood processing procedure with the adjusted parameter configuration that has been selected.

Aspect 17. The blood processing device of any one of the preceding Aspects, further comprising a database including information regarding a response of the subject to previous use of the blood processing device for the subject, wherein the controller is programmed to retrieve said information regarding the medical profile of the subject from the database.

Aspect 18. The blood processing device of any one of the preceding Aspects, further comprising a database including information regarding a result of previous use of the blood processing device for the subject, wherein the controller is programmed to retrieve said information regarding the medical profile of the subject from the database.

Aspect 19. The blood processing device of any one of the preceding Aspects, further comprising a database including information regarding an adjustment made to a procedural parameter during previous use of the blood processing device for the subject, wherein the controller is programmed to retrieve said information regarding the medical profile of the subject from the database.

Aspect 20. The blood processing device of any one of the preceding Aspects, further comprising a display screen, wherein the controller is programmed to determine at least one possible adjustment to a parameter of a blood processing procedure to be executed for the subject prior to the controller executing the blood processing procedure, control the display screen to display said at least one possible adjustment to said parameter of the blood processing procedure to be executed for the subject, and execute the blood processing procedure for the subject with said at least one possible adjustment to said parameter only after an operator has approved of said at least one possible adjustment to said parameter.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood processing device (10), comprising:
a pump system (18) configured to be actuated during a blood processing procedure to draw blood from a subject and/or to convey a fluid to the subject; and
a controller (20) programmed to control operation of the pump system (18) during the blood processing procedure, wherein the controller (20) is further programmed to
retrieve or be provided with information regarding a medical profile of the subject,
adjust at least one parameter of the blood processing procedure based at least in part on the medical profile of the subject, and
execute the blood processing procedure with said at least one adjusted parameter.

2. The blood processing device (10) of claim 1, wherein the medical profile of the subject includes a previously indicated preference of the subject, a physical characteristic of the subject, and/or a physical characteristic of the blood of the subject.

3. The blood processing device (10) of any one of the preceding claims, wherein the medical profile of the subject includes information regarding a response of the subject to execution of a previous iteration of the blood processing procedure and/or information regarding a result of execution of a previous iteration of the blood processing procedure for the subject and/or information regarding an adjustment made to a procedural parameter for a previous iteration of the blood processing procedure executed for the subject.

4. The blood processing device (10) of any one of the preceding claims, wherein the medical profile of the subject includes information regarding a response of the subject to execution of a different type of blood processing procedure and/or information regarding a result of execution of a different type of blood processing procedure for the subject and/or information regarding an adjustment made to a procedural parameter for a different type of blood processing procedure executed for the subject.

5. The blood processing device (10) of any one of the preceding claims, wherein the controller (20) is programmed to
adjust said at least one parameter of the blood processing procedure directly based on the medical profile of the subject,
adjust a second parameter of the blood processing procedure based on adjustment of said at least one parameter of the blood processing procedure, and
execute the blood processing procedure with said adjusted parameters.

6. The blood processing device (10) of any one of the preceding claims, wherein the controller (20) is programmed to retrieve said information regarding the medical profile of the subject from a data management system or a blood establishment computer system (42) or to be provided with said information regarding the medical profile of the subject from the data management system or the blood establishment computer system (42).

7. The blood processing device (10) of any one of the preceding claims, further comprising a display screen (46), wherein the controller (20) is programmed to control the display screen (46) to display a prompt for an operator to enter said information regarding the medical profile of the subject.

8. The blood processing device (10) of any one of the preceding claims, wherein the controller (20) is programmed to
execute a plurality of different types of blood processing procedures, and
prevent execution of at least one of said plurality of different types of blood processing procedures based on the medical profile of the subject.

9. The blood processing device (10) of any one of the preceding claims, wherein the controller (20) is programmed to
determine a plurality of possible adjusted parameter configurations for executing the blood processing procedure for the subject,
select one of the possible adjusted parameter configurations based on a required time to complete the blood processing procedure for the subject, and
execute the blood processing procedure with the adjusted parameter configuration that has been selected.

10. The blood processing device (10) of any one of the preceding claims, wherein the controller (20) is programmed to
determine a plurality of possible adjusted parameter configurations for executing the blood processing procedure for the subject,
select one of the possible adjusted parameter configurations based on a comfort level for the subject during the blood processing procedure, and
execute the blood processing procedure with the adjusted parameter configuration that has been selected.

11. The blood processing device (10) of any one of the preceding claims, wherein the controller (20) is programmed to
determine a plurality of possible adjusted parameter configurations for executing the blood processing procedure for the subject,
select one of the possible adjusted parameter configurations based on an operational efficiency of a facility for which the blood processing procedure is to be executed, and
execute the blood processing procedure with the adjusted parameter configuration that has been selected.

12. The blood processing device (10) of any one of the preceding claims, further comprising a database (44) including information regarding a response of the subject to previous use of the blood processing device (10) for the subject, wherein the controller (20) is programmed to retrieve said information regarding the medical profile of the subject from the database (44).

13. The blood processing device (10) of any one of the preceding claims, further comprising a database (44) including information regarding a result of previous use of the blood processing device (10) for the subject, wherein the controller (20) is programmed to retrieve said information regarding the medical profile of the subject from the database (44).

14. The blood processing device (10) of any one of the preceding claims, further comprising a database (44) including information regarding an adjustment made to a procedural parameter during previous use of the blood processing device (10) for the subject, wherein the controller (20) is programmed to retrieve said information regarding the medical profile of the subject from the database (44).

15. The blood processing device (10) of any one of the preceding claims, further comprising a display screen (46), wherein the controller (20) is programmed to
determine at least one possible adjustment to a parameter of a blood processing procedure to be executed for the subject prior to the controller (20) executing the blood processing procedure,
control the display screen (46) to display said at least one possible adjustment to said parameter of the blood processing procedure to be executed for the subject, and
execute the blood processing procedure for the subject with said at least one possible adjustment to said parameter only after an operator has approved of said at least one possible adjustment to said parameter.
